# EUROPÄISCHE PATENTSCHRIFT

(11) **EP 1 258 235 B1**
(45) Veröffentlichungstag und Bekanntmachung des Hinweises auf die Patenterteilung: **31.03.2004**
(21) Anmeldenummer: 02008832.4
(22) Anmeldetag: 19.04.2002
(51) Int. Cl.: A61K 7/021, A61K 7/02, C09D 13/00

(54) **Puderstiftmine oder Puderkreide**
Powder pencil core or powder chalk
Mine de crayon en poudre ou craie en poudre

(30) Priorität: 19.04.2001 DE 10119292
(43) Veröffentlichungstag der Anmeldung: 20.11.2002
(73) Patentinhaber: Faber- Castell AG, 90546 Stein (DE)
(72) Erfinder: Appel, Reiner, 90522 Oberasbach (DE); von Godin, Harald, 90522 Oberasbach (DE); Kinzel, Joachim, 90547 Stein (DE); Lugert, Gerhard, Dr., 90431 Nürnberg (DE)
(74) Vertreter: Mörtel & Höfner

(56) Entgegenhaltungen:
- DE-B- 2 540 877
- US-A- 5 030 446
- "CTFA 2000 International buyers' guide, Volume 2" 2000 , THE COSMETIC, TOILETRY, AND FRAGRANCE ASSOCIATION XP002210594 * Seite 633, linke Spalte, Zeile 16 - Zeile 20 *
- SÜD-CHEMIE: "Associative Thickeners" [Online] XP002209829 Gefunden im Internet: <URL: http://www.rheologicals.com/per-aqueous.sh tml> [gefunden am 2002-08-13] Applications
- "International Cosmetic Ingredient Dictionary and Handbook, Ninth Edition 2002, Volume 2 " 2002 , THE COSMETIC, TOILETRY, AND FRAGRANCE ASSOCIATION XP002210595 * Seite 1283, rechte Spalte, Absatz 2 *

## Beschreibung

Die Erfindung betrifft Puderstiftminen insbesondere für Kosmetikstifte und Puderkreiden. Letztere sind auch unter der Bezeichnung Softpastellkreiden bekannt. In DE 25 40 877 C2 ist ein Kosmetikstift mit einer Mine aus einem Formkörper mit poröser Struktur bestehend aus Glimmer, Magnesiummyristat als Haftvermittler und Bentonit beschrieben. Eine glimmer- und talkumhaltige Puderstiftmine, die als Bindemittel sphärisches Cellulose-Puder enthält, ist aus EP 0 432 279 A1 bekannt. Diese Minen besitzen wie gipshaltige Minen (US 4 624 273) oder trikalziumphosphathaltige Minen (DE 33 47 756 C2) ebenfalls eine poröse Struktur. Aufgrund ihrer geringen mechanischen Festigkeiten ist die Herstellung dieser Minen problematisch, da die Gefahr eines Minenbruches sehr groß ist. Nachteilig ist weiterhin, dass die Minen auch bei der Applikation auf der Haut und beim Spitzen leicht brechen und zum Bröseln neigen.

Davon ausgehend ist es Aufgabe der Erfindung eine Mine für Puderstifte und Puderkreiden für kosmetische Zwecke oder auch für Pastellzeichnungen auf Papier oder anderen Oberflächen vorzuschlagen, die sich durch eine erhöhte Festigkeit und Flexibilität während ihrer Herstellung, eine sehr weiche, nicht bröselnde Puderabgabe beim Applizieren und eine gute Spitzbarkeit mit üblichen Spitzgeräten auszeichnet.

Diese Aufgabe wird dadurch gelöst, dass als Verdicker ein Copolymer verwendet wird, das ein Polyoxyethylenglykol-Polymer (PEG) mit 5 bis 300 CH₂-CH₂-O-Monomeren und Tetramethoxymethylglycouril-Monomere enthält. Vorzugsweise wird ein Verdicker eingesetzt, der ein Copolymer aus PEG-180 (q.v.), Dodoxynol-5, PEG-25-tristyrylphenol und Tetramethoxymethylglycouril-Monomeren ist. Dieser Stoff ist nach der INCI-Nomenklatur mit "Polyether-1" bezeichnet. Bei dem erwähnten Copolymerbestandteil Dodoxynol-5 handelt es sich um PEG-5 Dodecyl-phenylether. Überraschender Weise kann durch die Verwendung solcher Assoziativverdicker nicht nur eine Verdickung und emulgierende Wirkung der während der Herstellung mit Wasser versetzten pulverförmigen Bestandteile, sondern auch innerhalb der Herstellung eine deutlich verbesserte Flexibiliät der extrudierten Minenstränge sowie eine gleichmäßige gute Extrudierbarkeit im Vergleich zum bisherigen Stand der Technik beobachtet werden, wobei Festigkeiten in der Größenordung von spröden, unflexiblen Vergleichsminen mit Bentone (Beispiel V)erreicht werden. Bei einem sehr geringen Zusatz von organischen Bindemitteln wie z. B. Sodium Alginate kann nahezu eine Verdopplung der Bruchfestikeit erreicht werden, ohne dass die mechanischen Eigenschaften, wie sehr weiche Applizierbarkeit, ausgezeichnete Spitzbarkeit und Flexibilität des zunächst wasserhaltigen Minenstranges, beeinträchtigt werden.
Als pulverhaltige Füllstoffe eignen sich insbesondere Polymethylmethacrylate und Mica. Vorzugsweise wird Mica verwendet, dem ein geringer Anteil Silica (0,5 bis 7 Gew.%) zugesetzt ist. Es hat sich überraschenderweise herausgestellt, dass sich Mischungen der genannten Füllstoffe durch eine sehr weiche, pudrige Abgabe auszeichnen und problemlos herstellbar und sind. Die mit Wasser vermischten Ausgangsstoffe, im einfachsten Fall also Verdicker und Füllstoffe, lassen sich zu Massen mit einer homogenen Verteilung der Füllstoffpartikel verarbeiten, wobei die Massen ihre Konsistenz auch während des Extrusionsvorgangs beibehalten, d.h. eine Trennung zwischen der Verdicker-Phase und der Feststoffphase findet nicht statt.

Allgemein gesprochen wird eine Mischung aus einem Füllstoff mit im Wesentlichen kugeligen Partikeln und einem Füllstoff mit plättchenförmigen Partikeln verwendet. Besonders gute Ergebnisse werden mit Kaliglimmer (Muskovit) erzielt.

Geringe Zusätze (< 10 %) von Ton, Kaolin, Talkum, Magnesiummyristat oder Calciumstearat) können insbesondere auf die Abgabe der Masse bei Applikation und die mechanische Festigkeit Einfluß nehmen. Als Hilfstoffe beim Auspressen der Minen und Haftvermittler sind Netzmittel, wie z B. Disodium-Laureth-Sulfosuccinate (INCI-Name), geeignet. Zur Verbesserung der Gleitfähigkeit während der Applikation und Verbesserung der Haftfestigkeit des Puders sowie zur Verringerung von Rißbildungen der Minen beim Trocknen können in geringen Mengen (< 10 %) Wachse, Fette, Öle (z. B. Castoroil) oder Emulgatoren zugesetzt werden. Exemplarisch wird Hydrogenated Palm Kernel Glycerides, Hydrogenated Palm Glycerides angeführt. Der Zusatz von pflegenden Substanzen wie Lanolin, Aloe Vera, Tocopherol u. a. sowie Konservierungsmittel oder Aromen ist möglich.

Im folgenden wird eine Rahmenrezeptur angegeben, die Basis für die Herstellung von Puderminen und Puderkreiden ist:

| Rahmenrezeptur: | |
|---|---|
| Verdicker | 0,1 - 5 Gew.% |
| Organisches Bindemittel | 0 - 3 Gew.% |
| Füllstoffe | 40 - 95 Gew.% |
| Netzmittel | 0 - 10 Gew.% |
| Wachse, Fette, Öle, Emulgatoren | 0 - 10 Gew.% |
| Farbmittel | 0 - 50 Gew.% |
| Additive (z. B. Aromen, Konservierungsmittel, pflegende Substanzen) | 0 - 5 Gew.% |

Eine Mine oder Kreide, die nur die "Muss-Bestandteile" der Rahmenrezeptur enthält, weist folgende Zusammensetzung auf:

| Beipiel 1: | |
|---|---|
| Poyether-1 | 3,0 Gew.% |
| Mica | 69,0 Gew.% |
| PMMA | 28,0 Gew.% |

Auf der Grundlage dieser Rahmenrezeptur wurden Minen für kosmetische Anwendungen auf folgende Weise hergestellt:
Die pulverförmigen Bestandteile wurden in einem Kneter oder Mischer unter Zugabe von Wasser in einer Menge von 20 - 40 Gew.% gleichmäßig vermischt. Die homogene Masse wurde anschließend zu einem Strang mit einem Durchmesser von 6 mm extrudiert und abgelängt. Die so erhaltenen Minenabschnitte wurden bei bei milder Temperatur (20-70°C) getrocknet. Auf diese Weise wurden drei verschieden Minenmassen bzw. Minen und ein Vergleichsmine (Beispiel V) hergestellt. Die jeweilige Minenzusammensetzung und einige charakteristische Eigenschaften gehen aus der folgenden Tabelle hervor.

| Rohmaterial | Beispiel V Vergleich Schwarze Mine [Gew.Teil e] | Beispiel 2 Schwarze Mine [Gew.Teil e] | Beispiel 3 Schwarze Mine [Gew.Teil ] | Beispiel 4 Gelbe Mine [Gew.Teil e] |
|---|---|---|---|---|
| Polyether 1 (Pure Thix® TX-1442, SÜD-CHEMIE) | - | 3,0 | 3,0 | 1 |
| Sodium Alginate (E 401) | - | - | 0,3 | 0,3 |
| Bentone EW (RHEOX, INC) | 3,0 () | - | - | - |
| Mica 97 - 99 %, Silica 1 - 3 % (CAS 12001-26-2, 7631-86-9) | 20,0 () | 20,0 | 20,0 | 30,0 |
| Polymethylmethacrylate | 40,0 | 40,0 | 40,0 | 50,0 |
| Disodium Laureth Sulfosuccinate | 5,0 | 5,0 | 5,0 | 6,0 |
| Hydrogenated Palm Kernel Glycerides, Hydro-genated Palm Glycerides (CAS 67701-26-2) | 4,0 | 4,0 | 4,0 | - |
| Eisenoxydschwarz CI 77499 | 30,0 | 30,0 | 30,0 | - |
| Titandioxyd CI 77891 | - | | | 15,0 |
| FD & C Yellow No. 5. Al Lake CI 19140:1 | - | | | 2,4 |
| Eisenoxydgelb CI 77492 - 77491 | - | | | 0,5 |
| | | | | |
| Bruchfestigkeit der trockenen Mine [N] bei 14,8mm Auflageabstand der Mine | 6 | 5 | 9 | 6 |
| Extrudierbarkeit | Schlecht | Gut | Gut | Gut |
| Flexibilität der frisch extrudierten Mine | Schlecht | Hoch | Hoch | Hoch |

Selbstverständlich lassen sich auf Basis der oben angegebenen Rahmenrezeptur Minen oder Kreiden mit kleineren oder größeren Durchmessern als 6 mm herstellen. Denkbar sind Minen und Kreiden mit Durchmessern etwa von 3 bis 12 mm.

## Patentansprüche

1. Puderstiftmine oder Kreide mit einem Füllstoff und einem Verdicker,
**dadurch gekennzeichnet,**
**dass** als Verdicker ein Copolymer aus wenigstens einem Polyoxyethylenglykol-Polymer (PEG) mit 5 bis 300 CH₂-CH₂-O- Monomeren und Tetramethoxymethylglycouril-Monomeren enthalten ist.

2. Puderstiftmine oder Kreide nach Anspruch 1,
**dadurch gekennzeichnet,**
**dass** ein Copolymer aus PEG-180, Dodoxynol-5, PEG-25-tristyrylphenol und Tetramethoxymethylglycouril-Monomeren enthalten ist.

3. Puderstiftmine oder Kreide nach Anspruch 2
**dadurch gekennzeichnet,**
**dass** Polyether-1 enthalten ist.

4. Puderstiftmine oder Kreide nach Anspruch 1,2 oder 3,
**dadurch gekennzeichnet,**
**dass** als Füllstoff Partikel aus PMMA enthalten sind.

5. Puderstiftmine oder Kreide nach einem der Ansprüche 1 - 4
**dadurch gekennzeichnet,**
**dass** als weiterer Füllstoff Mica enthalten ist.

6. Puderstiftmine oder Kreide nach einem der Ansprüche 1 - 5
**dadurch gekennzeichnet,**
**dass** als weiterer Füllstoff Silica enthalten ist.

7. Puderstiftmine oder Kreide nach einem der Ansprüche 1 - 6
**gekennzeichnet durch**
| folgende Rezeptur: | |
|---|---|
| Verdicker | 0,1 - 5 Gew.% |
| Organisches Bindemittel | 0 - 3 Gew.% |
| Füllstoffe | 40 - 95 Gew.% |
| Netzmittel | 0 - 10 Gew.% |
| Wachse, Fette, Öle, Emulgatoren | 0 - 10 Gew.% |
| Farbmittel | 0 - 50 Gew.% |
| Additive (z. B. Aromen, Konservierungsmittel, pflegende Substanzen) | 0 - 5 Gew.% |

8. Puderstiftmine oder Kreide nach Anspruch 7,
**dadurch gekennzeichnet,**
**dass** das Verhältnis von PMMA zu Mica oder zu einer Mischung aus Mica und Silica 35-45 zu 55-65 beträgt.

9. Puderstiftmine oder Kreide nach Anspruch 7 oder 8,
**dadurch gekennzeichnet,**
**dass** Silica mit einem Anteil 0,5 bis 7,0 Gew.%, bezogen auf die Gesamtmenge von Mica und Silica, enthalten ist.

10. Puderstiftmine oder Kreide nach einem der Ansprüche 1 bis 9,
**dadurch gekennzeichnet,**
**dass** ein organisches Bindemittel enthalten ist.

11. Puderstiftmine oder Kreide nach Anspruch 10,
**dadurch gekennzeichnet,**
**dass** Sodium-alginate enthalten ist.

12. Puderstiftmine oder Kreide nach einem der Ansprüche 1 bis 11,
**dadurch gekennzeichnet,**
**dass** als Netzmittel Disodium-Laureth-Sulfosuccinate enthalten ist.

13. Puderstiftmine oder Kreide nach einem der Ansprüche 1 bis 12,
**dadurch gekennzeichnet,**
**dass** als Fettsäurederivat Hydrogenated Palm Kernel Glycerides, Hydrogenated Palm Glycerides enthalten ist.

## Claims

1. Powder pencil core or chalk with a filler and a thickener,
**characterized in that**
the thickener present is a copolymer of at least one polyoxyethylene glycol polymer (PEG) with 5 to 300 CH₂-CH₂-O monomers and tetramethoxymethylglycouril monomers.

2. Powder pencil core or chalk according to Claim 1,
**characterized in that**
a copolymer of PEG-180, dodoxynol-5, PEG-25 tristyrylphenol and tetramethoxymethylglycouril monomers is present.

3. Powder pencil core or chalk according to Claim 2,
**characterized in that**
polyether-1 is present.

4. Powder pencil core or chalk according to Claim 1, 2 or 3,
**characterized in that**
the filler present is PMMA particles.

5. Powder pencil core or chalk according to one of Claims 1-4,
**characterized in that**
mica is present as further filler.

6. Powder pencil core or chalk according to one of Claims 1-5
**characterized in that**
silica is present as further filler.

7. Powder pencil core or chalk according to one of Claims 1-6
**characterized by**
the following formulation:
| | |
|---|---|
| thickener | 0.1 - 5% by weight |
| organic binder | 0 - 3% by weight |
| fillers | 40 - 95% by weight |
| wetting agents | 0 - 10% by weight |
| waxes, fats, oils, emulsifiers | 0 - 10% by weight |
| colorants | 0 - 50% by weight |
| additives (e.g. aromas, preservatives, care substances) | 0 - 5% by weight. |

8. Powder pencil core or chalk according to Claim 7,
**characterized in that**
the ratio of PMMA to mica or to a mixture of mica and silica is 35-45 to 55-65.

9. Powder pencil core or chalk according to Claim 7 or 8,
**characterized in that**
silica is present in an amount of from 0.5 to 7.0% by weight, based on the total amount of mica and silica.

10. Powder pencil core or chalk according to one of Claims 1 to 9,
**characterized in that**
an organic binder is present.

11. Powder pencil core or chalk according to Claim 10,
**characterized in that**
sodium alginate is present.

12. Powder pencil core or chalk according to one of Claims 1 to 11,
**characterized in that**
disodium laureth sulphosuccinate is present as wetting agent.

13. Powder pencil core or chalk according to one of Claims 1 to 12,
**characterized in that**
hydrogenated palm kernel glycerides, hydrogenated palm glycerides is present as fatty acid derivative.

## Revendications

1. Mine de crayon en poudre ou craie comprenant une charge et un épaississant,
**caractérisé en ce qu'**elle comprend comme épaississant un copolymère constitué d'au moins un polymère de polyoxyéthylèneglycol (PEG) ayant de 5 à 300 monomères de CH₂-CH₂-O- et de monomères de tétraméthoxyméthylglycouril.

2. Mine de crayon en poudre ou craie suivant la revendication 1,
**caractérisée en ce qu'**elle comprend un copolymère constitué de monomères de PEG-180, de dodoxynol -5, de PEG-25-tristyrylphénol et de tétraméthoxyméthylglycourile.

3. Mine de crayon en poudre ou craie suivant la revendication 2,
**caractérisée en ce qu'**elle comprend un polyétheroxyde - 1.

4. Mine de crayon en poudre ou craie suivant la revendication 1, 2 ou 3,
**caractérisée en ce qu'**elle comprend comme charge des particules de PMMA.

5. Mine de crayon en poudre ou craie suivant l'une des revendications 1 à 4,
**caractérisée en ce qu'**elle comprend comme charge supplémentaire du mica.

6. Mine de crayon en poudre ou craie suivant l'une des revendications 1 à 5,
**caractérisée en ce qu'**elle comprend comme charge supplémentaire de la silice.

7. Mine de crayon en poudre ou craie suivant l'une des revendications 1 à 6,
**caractérisée par** la composition suivante :
- Epaississant de 0, 1 à 5 % en poids
- Liant organique de 0 à 3 % en poids
- Charge de 40 à 95 % en poids
- Agent mouillant de 0 à 10 % en poids
- Cire, graisse, huile, émulsionnant, de 0 à 10 % en poids
- Agent colorant de 0 à 50 % en poids
- Additif ( par exemple aromatisant, conservateur, adjuvant) de 0 à 5 % en poids

8. Mine de crayon en poudre ou craie suivant la revendication 7,
**caractérisée en ce que** le rapport du PMMA au mica ou au mélange de mica et de silice est compris entre 35 - 45 et 55 - 65.

9. Mine de crayon en poudre ou craie suivant la revendication 7 ou 8,
**caractérisée en ce qu'**elle comprend de la silice en une proportion représentant de 0,5 à 7,0 % du poids total du mica et de la silice.

10. Mine de crayon en poudre ou craie suivant l'une des revendications 1 à 9,
**caractérisée en ce qu'**elle comprend un liant organique.

11. Mine de crayon en poudre ou craie suivant la revendication 10,
**caractérisée en ce qu'**elle contient de l'alginate de sodium.

12. Mine de crayon en poudre ou craie, suivant l'une des revendications 1 à 11,
**caractérisée en ce qu'**elle contient comme agent mouillant du laurylsulfosuccinate disodique.

13. Mine de crayon en poudre ou craie, suivant l'une des revendications 1 à 12,
**caractérisée en ce qu'**elle comprend comme dérivé d'acide gras, des glycérides de noix de palme hydrogénés, des glycérides de palme hydrogénés.
